# DEMANDE DE BREVET EUROPEEN

(11) **EP 0 739 888 A1**
(43) Date de publication de la demande: **30.10.1996**
(21) Numéro de dépôt: 96390003.0
(22) Date de dépôt: 22.04.1996
(51) Int. Cl.: C07D 317/36

(54) **Procédé de fabrication de carbonate de glycérol à partir de clycérol et d'un carbonate organique cyclique, en particulier carbonate d'éthylène ou de propylène**

(30) Priorité: 24.04.1995 FR 9504961
(71) Demandeur: ORGANISATION NATIONALE INTERPROFESSIONNELLE DES OLEAGINEUX- ONIDOL, 75008 Paris (FR)
(72) Inventeur: Mouloungui, Zéphirin, 31500 Toulouse (FR); Yoo, Jeong-Woo, 31500 Toulouse (FR); Gachen, Christian-Alain, 31500 Toulouse (FR); Gaset, Antoine, 31000 Toulouse (FR); Vermeersch, Georges, 75015 Paris (FR)
(74) Mandataire: Cabinet BARRE LAFORGUE & associés

(57) **Abrégé**

L'invention concerne un procédé de fabrication de carbonate de glycérol à partir de glycérol et d'un carbonate organique cyclique en particulier carbonate d'éthylène ou de propylène. Le glycérol et le carbonate organique cyclique sont amenés à réagir en les mettant en contact dans un milieu solvant essentiellement constitué par un carbonate organique, en particulier carbonate réactif de départ, carbonate de glycérol formé ou mélange des deux carbonates, en présence d'un catalyseur solide comportant des sites basiques, en particulier résines macroporeuses anioniques bicarbonatées ou hydroxylées, ou zéolites de type X ou Y tridimensionnelles. Le diol formé est soutiré du milieu au cours de la réaction afin de déplacer l'équilibre.

## Description

L'invention concerne un procédé de fabrication de carbonate de glycérol par réaction du glycérol et d'un carbonate organique cyclique, en particulier carbonate d'éthylène ou de propylène.

Le carbonate de glycérol est un intermédiaire de synthèse d'un grand nombre de composés (carbamates, éthers, esters, ...). La fabrication du carbonate de glycérol est effectuée depuis de très nombreuses années par transestérification, en faisant réagir du glycérol et du carbonate d'éthylène en catalyse homogène en présence d'une base alcaline à une température d'environ 125° C/135° C (brevet US 2.915.529). La réaction conduit à un mélange des réactifs de départ, de carbonate de glycérol, d'éthylène glycol et de la base utilisée comme catalyseur. La séparation du carbonate de glycérol pour l'isoler des autres composés et notamment du glycérol exige ensuite une neutralisation acide et une distillation à basse pression ; cette dernière opération est de mise en oeuvre délicate en raison de la présence du glycérol contaminé par les sels issus de la neutralisation. L'obtention d'un mélange des réactifs de départ, des composés formés et de la base, nécessitant une séparation du carbonate de glycérol par des opérations complémentaires délicates, constitue le défaut essentiel de ce type de procédé.

Le brevet japonais **JP-A-06 329 663** visé dans l'Abstract Database WPI AN 95-048834 décrit un procédé de fabrication de carbonate de glycérol qui utilise comme catalyseur un oxyde métallique, en particulier l'alumine. Ce type de catalyseur nécessite des conditions opératoires sévères : travail sous vide (30-40 mm de Hg), température relativement élevée (135-140° C). Ces conditions opératoires constituent un inconvénient grave qui sur le plan industriel obère le coût du procédé et surtout conduit à des risques de décomposition du carbonate de glycérol formé compte tenu de la plage de températures et de pressions utilisées.

On connaît par ailleurs d'autres réactions de transestérification qui sont mises en oeuvre en catalyse hétérogène en vue de faciliter la séparation du catalyseur. En particulier le brevet US 4.691.041 décrit un procédé de préparation de carbonate de diméthyle (DMC) en présence de résines anioniques ou de zéolites. Toutefois, ce procédé et la plupart des transestérifications réalisées en catalyse hétérogène exigent une mise en oeuvre sous pression (6 à 7 bars) et conduisent à un mélange des réactifs de départ et des composés formés. La mise en oeuvre sous pression rend ces procédés onéreux, et, en général, une ou des opérations finales délicates restent nécessaires, pour séparer le carbonate recherché, même si le catalyseur ne pose plus de difficultés d'extraction.

Par ailleurs, le brevet US 5.091.543 décrit une transestérification d'un carbonate acyclique (DMC) par réaction avec un diol en présence de résines anioniques pour former un carbonate cyclique dérivé du diol utilisé. Toutefois, cette réaction conduit à des produits secondaires de transestérification (monocarbonate de diol et biscarbonate de diol) et l'on obtient, ici encore, en fin de réaction un mélange de plusieurs produits : réactifs de départ, produits formés et produits secondaires de transestérification : il est nécessaire de procéder à des opérations finales de séparation. En outre, cette réaction est longue (environ 20 heures) et le taux de conversion médiocre (53 %).

La présente invention se propose de fournir un nouveau procédé de fabrication de carbonate de glycérol, par transestérification, en faisant réagir du glycérol et un carbonate organique cyclique, en particulier le carbonate d'éthylène ou le carbonate de propylène.

L'objectif essentiel de l'invention est d'obtenir le carbonate de glycérol, soit à concentration élevée n'exigeant pas, pour la plupart des applications, de purification supplémentaire, soit en mélange facile a purifier.

Un autre objectif est de fournir un procédé bénéficiant d'une très bonne sélectivité vis à vis du carbonate de glycérol et d'un rendement élevé.

Un autre objectif est de fournir un procédé dont les conditions de mise en oeuvre (température modérée, courte durée, pression atmosphérique...) conduisent à de faibles coûts du produit fabriqué.

A cet effet, le procédé conforme à l'invention pour fabriquer du carbonate de glycérol à partir de glycérol et de carbonate organique cyclique se caractérise en ce que le glycérol et le carbonate organique cyclique sont amenés à réagir en les mettant en contact dans un milieu solvant essentiellement constitué par un carbonate organique ou un mélange de carbonates organiques, en présence d'un catalyseur solide comprenant une résine macroporeuse anionique bicarbonatée ou hydroxylée, ou une zéolite de type X ou Y tridimensionnelle comportant des sites basiques, le diol formé étant soutiré du milieu au cours de la réaction.

Par "zéolite comportant des sites basiques", on entend aussi bien une zéolite possédant initialement de tels sites basiques par exemple sites OH⁻, HCO₃⁻ ..., qu'une zéolite susceptible d'acquérir de tels sites basiques avant ou au cours de la réaction, en particulier au contact d'une source de synthons CO₂ (provenant du carbonate de départ lui-même, de l'air, ...).

Les expérimentations ont montré que les caractéristiques de mise en oeuvre du procédé de l'invention (catalyseur hétérogène prévu, milieu solvant constitué par un carbonate organique, soutirage du diol formé) se combinaient pour conduire à l'obtention d'un carbonate de glycérol de concentration élevée, facile à 5 purifier le cas échéant, et immédiatement séparable du catalyseur solide par voie physique.

On a pu constater que la sélectivité de la réaction était supérieure à 99 %, et son rendement de l'ordre de 90 % pour des durées de réaction de l'ordre de l'heure. Ces résultats sont atteints à température modérée, avantageusement ajustée entre 50° C et 110° C.

Il est à noter que le diol qui est soutiré au cours de la réaction est un sous-produit intéressant dans diverses applications industrielles : en particulier il peut servir à fabriquer le carbonate organique cyclique qui sert de réactif de départ dans la présente invention.

Selon un mode de mise en oeuvre préféré du procédé, on utilise comme solvant un carbonate organique de même nature que le carbonate réactif de départ ou que le carbonate formé, ou bien un mélange de ces deux carbonates. En particulier, le procédé de l'invention peut être avantageusement mis en oeuvre en disposant, au début de la réaction, le glycérol dans un excès du carbonate organique cyclique choisi comme réactif de façon que ce composé joue initialement à la fois le rôle de réactif et de solvant, le carbonate de glycérol formé faisant ensuite fonction de co-solvant et de solvant.

Le carbonate d'éthylène et/ou de propylène sont de préférence utilisés comme carbonate réactif de départ car ils conduisent à une cinétique de réaction plus élevée.

Le procédé de l'invention peut être mis en oeuvre en réacteur semi-fermé où sont disposés initialement les réactifs de départ et le catalyseur solide. Dans ce cas, le procédé est de préférence mis en oeuvre dans les conditions suivantes : on dispose initialement dans le réacteur du carbonate organique cyclique en excès en proportion comprise entre 1,5 et 3 fois la proportion stoechiométrique par rapport au glycérol, le diol formé étant soutiré du réacteur au moins pendant la phase d'équilibre de la réaction.

Par "phase d'équilibre de réaction", on désigne la phase de déroulement normal de celle-ci à l'exclusion de la phase initiale au cours de laquelle les produits formés sont encore en faible quantité.

Le procédé peut être interrompu lorsque le glycérol est épuisé ou pratiquement épuisé. Une simple filtration physique permet de recueillir un mélange carbonate initial/carbonate de glycérol qu'il est facile de séparer par une simple distillation (rendue facile par l'absence de glycérol). Il est également possible, et dans la plupart des cas avantageux, d'ajouter du glycérol au cours de la phase d'équilibre de la réaction de façon à obtenir un épuisement sensiblement concomitant du carbonate cyclique initial et du glycérol (le carbonate de glycérol formé jouant pratiquement seul le rôle de solvant au cours de la phase finale de la réaction). On obtient ainsi un carbonate de glycérol concentré, qui ne nécessite aucune purification dans la plupart des applications.

Le procédé de l'invention peut également être mis en oeuvre en continu dans un réacteur ouvert ou dans une colonne. Dans ce cas, le procédé est de préférence mis en oeuvre dans les conditions suivantes :
- pendant une phase initiale, on délivre dans le réacteur ouvert ou la colonne un mélange réactif comportant du carbonate organique cyclique en excès en proportion comprise entre 1,5 et 3 fois la proportion stoechiométrique par rapport au glycérol,
- en sortie de réacteur ou de colonne, on sépare le diol et le carbonate de glycérol formés,
- on réinjecte au moins partiellement le carbonate de glycérol formé dans le réacteur ou la colonne,
- pendant une phase de fonctionnement permanent, on délivre dans le réacteur ou la colonne un mélange réactif comportant le carbonate organique cyclique et le glycérol en proportions sensiblement stoechiométriques, le carbonate de glycérol formé étant toujours au moins partiellement réinjecté dans le réacteur ou la colonne.

Un tel procédé permet de fabriquer du carbonate de glycérol très concentré dans de remarquables conditions d'économie. Dans le cas d'un réacteur ouvert, la réaction se produit en milieu agité dans le réacteur contenant le catalyseur solide en suspension. Dans le cas d'une colonne, les réactifs sont amenés à percoler à travers un lit de catalyseur solide disposé dans la colonne.

Si l'on désire fabriquer des produits de pureté élevée, on utilisera de préférence comme réactif de départ du glycérol à 99 %. Dans le cas contraire, on peut utiliser de la glycérine à 50-65 % de glycérol (voisine par exemple de la transestérification des huiles de colza aux fins de fabrication d'esters méthyliques carburants).

Le procédé de l'invention est illustré par les exemples de mise en oeuvre qui suivent.

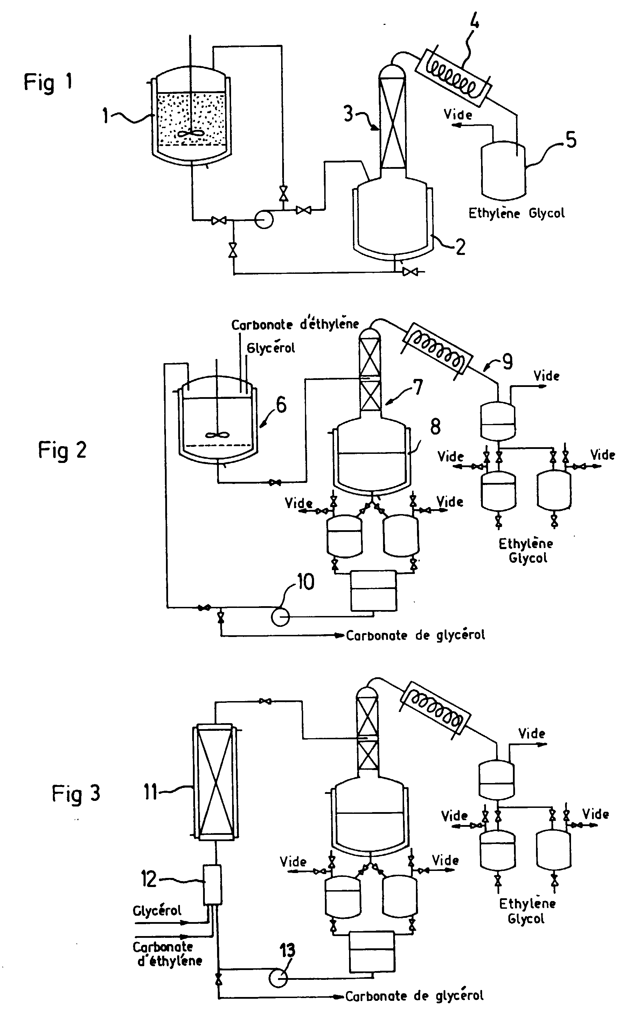

Les exemples 1 à 21 ont été mis en oeuvre en réacteur semi-fermé au moyen d'une installation telle que représentée schématiquement sur les dessins à la figure 1.

L'exemple 22 a été mis en oeuvre en continu dans un réacteur ouvert au moyen d'une installation telle que représentée schématiquement à la figure 2.

Les exemples 23 et 24 ont été mis en oeuvre en continu dans une colonne à lit fixe au moyen d'une installation telle que schématiquement représentée à la figure 3.

L'installation schématisée à la figure 1 permet une mise en oeuvre discontinue du procédé de l'invention.

Dans un réacteur semi-fermé agité, à double enveloppe 1, on introduit les quantités indiquées dans les exemples 1 à 22 de carbonate d'éthylène, de glycérol et de catalyseur sous forme solide granulaire. La réaction s'effectue à une température T et à la pression atmosphérique.

Au bout d'un temps t₁, un premier équilibre chimique est atteint à 87 % de carbonate de glycérol. Pour atteindre des concentrations supérieures on soutire alors l'éthylène glycol formé.

A cet effet, on envoie une partie ou la totalité du mélange réactionnel 2 dans un bouilleur associé à une colonne à distiller 3 travaillant sous basse pression (20 mb) jusqu'à élimination totale de l'éthylène glycol à travers un condenseur 4 vers un conteneur de récupération 5. Une fois cette opération terminée, on réintroduit par des moyens de canalisation et vannes classiques, le mélange restant (carbonate d'éthylène, glycérol, carbonate de glycérol) dans le réacteur 1. La réaction s'effectue jusqu'à un deuxième équilibre.

La réaction est pratiquement totale lorsqu'on a effectué trois cycles.

L'installation schématisée à la figure 2 permet une mise en oeuvre continue en réacteur ouvert (exemple 22).

Le procédé passe alors par une étape préalable correspondant à un cycle du procédé discontinu décrit plus haut. Cette étape peut le cas échéant, être supprimée grâce à une mise en place initiale de carbonate de glycérol dans le réacteur ouvert 6.

Lorsque cette étape préalable est terminée, le mélange réactionnel est envoyé en continu dans une colonne à distiller sous vide 7 associée à un bouilleur 8. L'éthylène glycol est soutiré en tête de colonne par un dispositif à condensation 9. Le pied de colonne composé de carbonate d'éthylène, de glycérol et de carbonate de glycérol est renvoyé en continu dans le réacteur ouvert 6 par une pompe 10. Cette étape dure jusqu'à ce que le pied de colonne ne soit pratiquement constitué que de carbonate de glycérol.

A partir de ce moment, on ajoute dans le réacteur 6 les réactifs en continu et on soutire en continu le carbonate de glycérol à la base du bouilleur 8.

L'installation schématisée à la figure 3 est similaire à celle de la figure 2 mais la réaction est effectuée dans une colonne 11 à lit fixe où les réactifs sont amenés à percoler. Ils sont préalablement mélangés dans un mélangeur 12 dans lequel peut être envoyée une partie du carbonate de glycérol formé par une pompe 13.

Il est à noter que, dans tous les exemples, on utilise comme réactif de départ du glycérol du commerce à 99%.

### Préparation de la résine pour les exemples 1-13 :

La résine anionique macroporeuse forte "Amberlyst A26" (marque déposée) Rohm et Haas livrée humide sous forme OH⁻ est fonctionnalisée sous la forme HCO₃⁻ de la manière suivante. Un volume de résine, préalablement gonflée d'eau déminéralisée est introduit dans une colonne munie d'un fritté. Le lavage à l'eau suivi d'une percolation d'une solution de 10 % de bicarbonate de potassium permet de réaliser l'échange d'anions OH⁻ contre HCO₃⁻. Ensuite, la résine est débarrassée de l'excédent de KHCO₃ avec de l'eau déminéralisée jusqu'à pH neutre. Un rinçage à l'éthanol puis à l'éther éthylique permet d'éliminer l'eau résiduelle. Le séchage final de la résine se fait sous vide. Cette résine sera désignée plus loin par l'expression : "résine Amberlyst A26 (HCO₃⁻)".

### EXEMPLE 1 : Préparation du carbonate de glycérol par réaction du glycérol et du carbonate d'éthylène en présence de la résine Amberlyst A26(HCO₃⁻) dans un réacteur semi-fermé.

Dans le réacteur 1 de la figure 1 muni d'un réfrigérant et d'un agitateur mécanique, on mélange 11,0 g (0,12 mole) de glycérol, 21,1 g (0,24 mole) de carbonate d'éthylène et 1 g (2,84 meq HCO₃/g⁻) de résine Amberlyst A26(HCO₃⁻). On porte le mélange réactionnel à 80° C sous agitation mécanique pendant une heure. Au bout de ce temps, le milieu réactionnel est amené à température ambiante et analysé par chromatographie en phase gazeuse sur colonne capillaire Carbowax 20 M avec le tétraéthylèneglycol comme étalon interne. 9,6 g de glycérol sont transformés en 12,4 g de carbonate de glycérol soit un rendement de 87,7 % étant donné une sélectivité supérieure à 99 %. La composition du milieu final est la suivante :

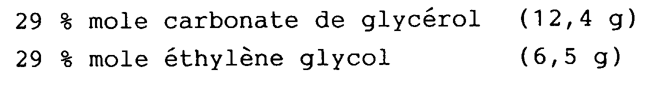

à 99%

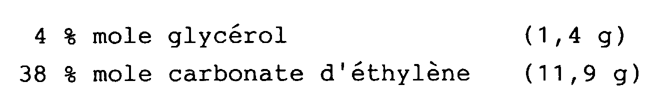

### EXEMPLE 2 : Préparation du carbonate de glycérol par réaction du glycérol et du carbonate de propylène en présence de la résine Amberlyst A26(HCO₃⁻) dans un réacteur semi-fermé.

Dans le réacteur 1 de la figure 1 muni d'un réfrigérant et d'un agitateur mécanique, on mélange 11,0 g (0,12 mole) de glycérol, 24,5 g (0,24 mole) de carbonate de propylène et 1 g (2,84 meq HCO₃/g⁻) de résine Amberlyst A26(HCO₃⁻). On porte le mélange réactionnel à 80° C sous agitation mécanique pendant 2 heures. Au bout de ce temps, le milieu réactionnel est amené à température ambiante et analysé par chromatographie en phase gazeuse sur colonne capillaire Carbowax 20 M avec le tétraéthylèneglycol comme étalon interne. 7,7 g de glycérol sont transformés en 8,6 g de carbonate de glycérol soit un rendement de 61 % étant donné une sélectivité supérieure à 99 %. La composition du milieu final est la suivante :

| | |
|---|---|
| 20 % mole carbonate de glycérol | (8,6 g) |
| 20 % mole propylène glycol | (4,5 g) |
| 13 % mole glycérol | (4,3 g) |
| 47 % mole carbonate de propylène | (14,7 g) |

### EXEMPLES 3-13 :

Une série d'essais de synthèse de carbonate de glycérol est réalisée en utilisant le réacteur semi-fermé de la figure 1 dans lequel le glycérol et le carbonate d'éthylène sont mélangés suivant les rapports massiques indiqués dans le tableau 1. Dans ces exemples, le catalyseur solide est la résine macroporeuse anionique Amberlyst A26 utilisée sous forme chlorure, hydroxyde ou bicarbonate. En présence de la résine Amberlyst A26(HCO₃⁻) on fait varier la quantité catalytique ainsi que la température de la réaction portée à 30° C, 50° C, 80° C et 110° C. Les résultats obtenus sont rassemblés dans le tableau 1. On peut noter que :
1) Quel que soit le contre ion anionique de la résine Amberlyst A26, la réaction est opérationnelle. La résine anionique est un catalyseur.
2) Dans les exemples 3, 5, 7, 8, 9 et 13, on observe une transformation élevée du glycérol en carbonate de glycérol avec des rendements en carbonate de glycérol supérieurs à 84,1 %.
3) Dans l'exemple 13, la résine Amberlyst A26(HCO₃⁻) permet une transformation quasi totale du glycérol (concentration résiduelle : 2 % mole) et donne un rendement de 90,4 % en carbonate de glycérol en un temps de réaction court (1 heure).
4) Dans l'exemple 13, le rôle solvant du carbonate d'éthylène et du carbonate de glycérol se traduit par une transformation élevée du glycérol.
5) Dans les exemples 3, 5, 7, 8, 9 et 13, la concentration molaire du carbonate de glycérol dans le mélange brut final est supérieure ou égale à 23 % alors que celle du glycérol est inférieure ou égale à 5 %.
6) Dans les exemples 5, 6 et 7, l'apport thermique est favorable à la réaction et un seuil de température (80°) semble être atteint.
7) Dans les exemples 8 et 9, on observe que 1 g de résine est la charge catalytique utile pour catalyser la transestérification du carbonate d'éthyle avec le glycérol.
8) Dans les exemples 8, 9 et 13, un effet de synergie dû à la charge catalytique et à la charge massique en carbonate organique est observé en faveur de la consommation du glycérol au cours de la transestérification du carbonate d'éthylène.

### EXEMPLES 14-20 :

5 Une série d'essais de synthèse de carbonate de glycérol est réalisée en utilisant le réacteur semi-fermé de la figure 1 dans lequel le glycérol et le carbonate d'éthylène sont mélangés en présence des zéolites de type x ou y tridimensionnel de formule chimique générale M_{x/n}(AlO₂)ₓ(SiO₂)_{y}.wH₂O.

### Prétraitement des zéolites

Le zéolite Na.X provient de la société Aldrich. Il est utilisé sans traitement préalable.

### Préparation des zéolites K, X

On introduit 10 g de zéolite sous forme Na dans 50 g de solution aqueuse de bicarbonate de potassium à 10 %. Après 10 heures de percolation, on remplace la solution liquide usagée par une solution fraîche. 10 heures après, on élimine la solution liquide, on lave les zéolites jusqu'à pH neutre. Les zéolites sont ensuite calcinés à 400° C pendant 3 heures. La montée et la descente de température se font lentement.

### Préparation de zéolite K, A

La procédure suivie est la même que celle utilisée pour le zéolite K, X, celui-ci étant remplacé par le zéolite A.

### Préparation de zéolite H, X

La procédure suivie est la même que celle utilisée pour le zéolite K, X, sauf que la solution de bicarbonate de potassium est remplacée par une solution aqueuse d'ammoniaque à 30 %.

### Préparation du carbonate de glycérol par réaction du glycérol et du carbonate d'éthylène en présence de zéolite

Dans le réacteur 1 de la figure 1 muni d'un réfrigérant et d'un agitateur mécanique on mélange 11,0 g (0,12 mole) de glycérol, 21,1 g (0,24 mole) de carbonate d'éthylène et 1 g de zéolite. On porte le mélange réactionnel à 80° C sous agitateur mécanique pendant 2 à 3 heures. Au bout de ce temps, le milieu réactionnel est amené à température ambiante et analysé par chromatographie en phase gazeuse sur colonne capillaire Carbowax 20 M avec le tétraéthylèneglycol comme étalon interne. Les résultats obtenus sont rassemblés dans le Tableau 2.

On note que les exemples 15, 16, 17 et 18 cumulent les meilleurs résultats avec une concentration molaire résiduelle de glycérol de 6-9 %, une concentration molaire élevée du carbonate de glycérol 25-27 % assortie d'un rendement meilleur en carbonate de glycérol de 74,2 à 81,1 %.

### EXEMPLE 21 : Préparation du carbonate de glycérol par réaction du glycérol avec le carbonate d'éthylène en présence de l'alumine sous forme γ-Al₂O₃ dans le réacteur semi-fermé.

Dans le réacteur 1 de la figure 1 muni d'un réfrigérant et d'un agitateur mécanique, on mélange 11,0 g (0,12 mole) de glycérol, 21,1 g (0,24 mole) de carbonate d'éthylène et 1 g de γ-Al₂O₃. On porte le mélange réactionnel à 80° C sous agitation mécanique pendant 2 heures. Au bout de ce temps, le milieu réactionnel est amené à température ambiante et analysé par chromatographie en phase gazeuse sur colonne capillaire Carbowax 20 M avec le tétraéthylène glycol comme étalon interne. 6,8 g de glycérol sont transformés en 8,8 g de carbonate de glycérol soit un rendement de 62,2 g étant donné une sélectivité supérieure de 99 %. La composition du milieu final est la suivante :

| | |
|---|---|
| 21 % mole carbonate de glycérol | (8,8 g) |
| 21 % mole éthylène glycol | (4,6 g) |
| 12 % mole glycérol | (4,2 g) |
| 46 % mole carbonate éthylène | (14,6 g) |

### EXEMPLE 22 : Préparation du carbonate de glycérol par réaction du glycérol avec le carbonate d'éthylène en présence de la résine Amberlyst A26(HCO₃⁻) dans un réacteur ouvert.

23,0 g (0,25 mole) de glycérol, 44,0 g (0,50 mole) de carbonate d'éthylène et 3 g (8,52 meq HCO₃/g⁻) de résine Amberlyst A26(HCO₃⁻) sont mélangés dans le réacteur 6 (figure 2) muni d'un système d'agitation et relié à une colonne de distillation sous vide 7. On porte le mélange réactionnel à 80° C sous agitation mécanique pendant 1 heure. Pendant la réaction, le mélange réactionnel est envoyé en continu dans la colonne 7 pour fractionner et séparer sous vide l'éthylène glycol qui est ensuite condensé par le condensateur 9 et recueilli dans les récupérateurs D₁, D₂ et D₃. La base du bouilleur 8 est alors composée essentiellement de carbonate de glycérol, glycérol et carbonate d'éthylène qui est renvoyé dans le réacteur ouvert 6 par une pompe 40. Cette opération est répétée 3 fois et dure 2 heures. Au bout de ce temps, le milieu réactionnel est amené à température ambiante et analysé par chromatographie en phase gazeuse sur colonne capillaire Carbowax 20 M avec le tétraéthylèneglycol comme étalon interne. 21,6 g de glycérol sont transformés en 27,7 g de carbonate de glycérol soit un rendement de 93,9 % étant donné que la sélectivité est supérieure à 99 %. La composition du milieu final est la suivante :

| | |
|---|---|
| 86,2 % mole carbonate de glycérol | (27,7 g) |
| 1,5 % mole éthylène glycol | (0,2 g) |
| 5,5 % mole glycérol | (1,4 g) |
| 6,8 % mole carbonate d'éthylène | (1,7 g) |

### EXEMPLE 23 : Préparation du carbonate de glycérol par réaction du glycérol avec le carbonate d'éthylène en présence de la résine Amberlyst A26(HCO₃⁻) dans une colonne à lit fixe

23,0 g (0,25 mole) de glycérol, 44,0 g (0,50 mole) de carbonate d'éthylène sont percolés à travers un lit de 3 g (8,52 meq HCO₃⁻/g) Amberlyst A26(HCO₃⁻) disposé dans une colonne de verre 11 (diamètre interne 20 mm, hauteur : 150 mm) (figure 3). La colonne 11 est thermostatée et portée à 80° C. Elle est reliée à une colonne à distiller sous vide destinée à fractionner et à séparer l'éthylène glycol que l'on recueille après condensation dans les récupérateurs D₁, D₂ et D₃. A la base du bouilleur (B), on soutire en continu le mélange résiduaire composé essentiellement de carbonate de glycérol, glycérol et carbonate d'éthylène que l'on renvoie dans la colonne 11 au moyen de la pompe 13. Cette opération est répétée 3 fois. Chaque cycle dure 30 minutes après élimination de l'éthylène glycol. Au bout de cette étape continue, le milieu réactionnel est amené à température ambiante et analysé par chromatographie en phase gazeuse sur colonne capillaire Carbowax 20 M avec le tétraéthylèneglycol comme étalon interne. 22,1 g de glycérol sont transformés en 30,3 g de carbonate de glycérol soit un rendement de 99,1 % étant donné une sélectivité supérieure à 99 %. La composition du milieu final est la suivante :

| | |
|---|---|
| 96,2 % mole carbonate de glycérol | (30,3 g) |
| 0,0 % mole éthylène glycol | |
| traces % mole glycérol | |
| 3,5 % mole carbonate d'éthylène | (0,8 g) |

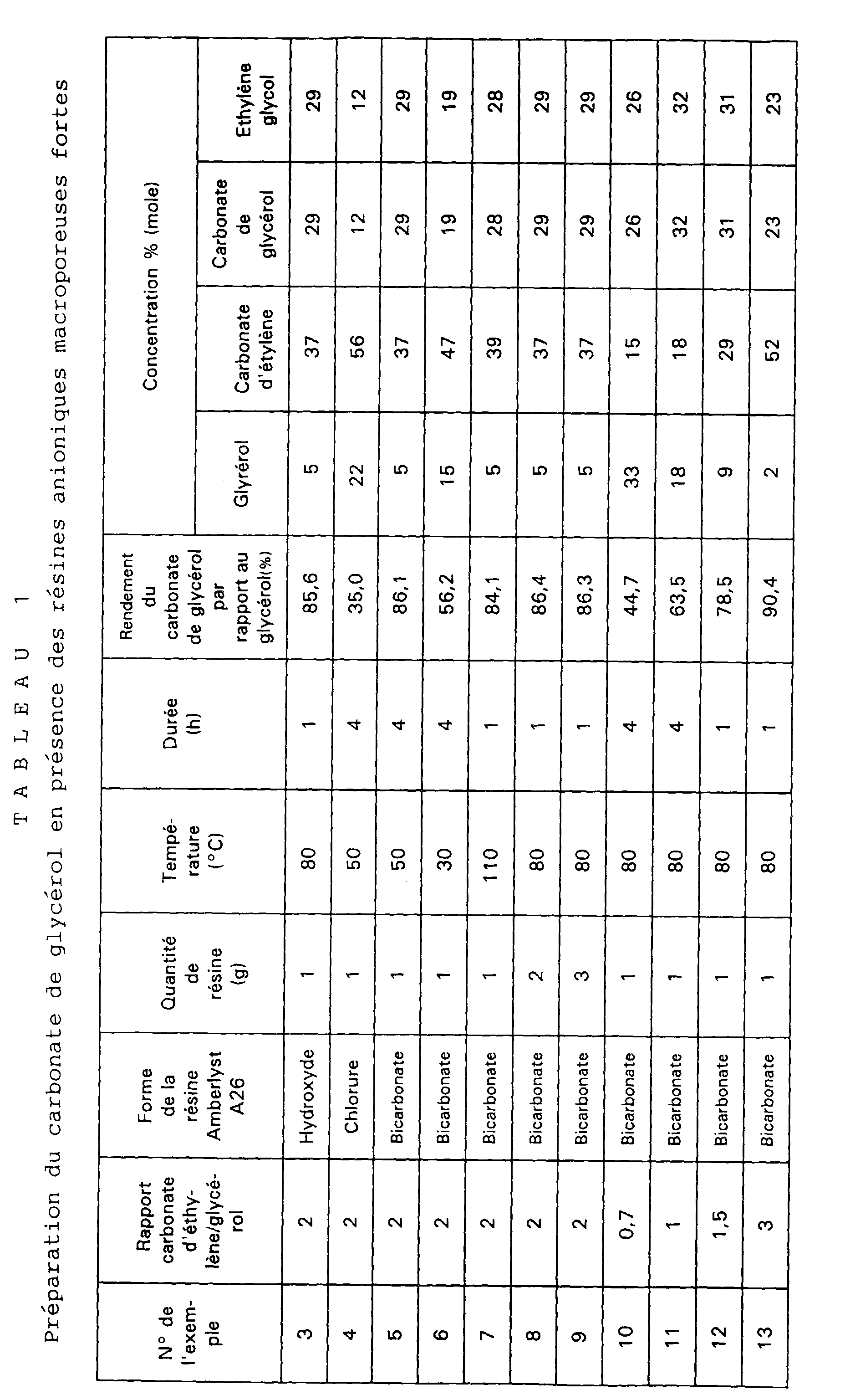

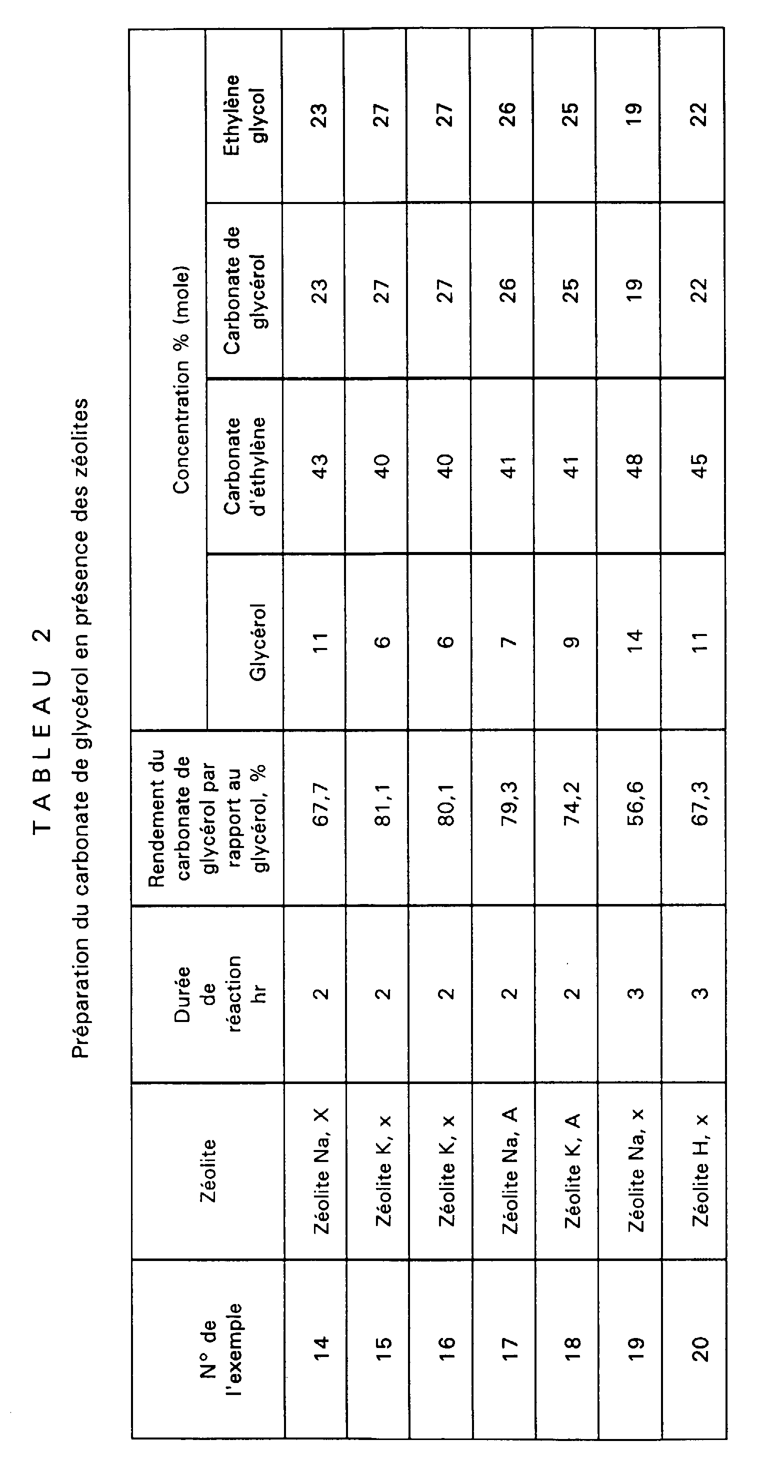

### EXEMPLE 24 : Préparation du carbonate de glycérol par réaction du glycérol avec le carbonate d'éthylène en présence de la résine Amberlyst A26(HCO₃⁻) dans un lit fixe en milieu carbonate de glycérol

On prend 64 g d'une solution concentrée de carbonate de glycérol préalablement préparée (59 g de carbonate de glycérol, 1 g de glycérol et 4 g de carbonate d'éthylène) auxquels on ajoute 36 g d'un mélange stoechiométrique de glycérol (18,6 g) et de carbonate d'éthylène (17,4 g). Cet ensemble est percolé pendant 30 minutes à travers un lit fixe de 3 g de résine Amberlyst A26(HCO₃⁻)(8,52 meq HCO₃⁻/g) disposés dans une colonne de verre 11 (diamètre = 20 mm, hauteur = 150 mm) (figure 3). La colonne 11 est thermostatée et portée à 80° C. Elle est reliée à une colonne à distiller sous vide destinée à fractionner et à séparer l'éthylène glycol que l'on recueille après condensation dans les récupérateurs D₁, D₂ et D₃. A la base du bouilleur (B), on soutire le mélange résiduaire composé de carbonate de glycérol (81,3 % masse), de glycérol (9,9 %), de carbonate d'éthylène (6,5 %) et d'éthylène glycol (2,0 %). 25% de ce mélange sont stockés et, les 75 % restant sont renvoyés dans la colonne à lit fixe de résine après addition de 36 g d'un mélange stoechiométrique de carbonate de glycérol (18,6 g) et de glycérol (17,4 g). Ce cycle est répété 6 fois toujours dans les mêmes conditions que précédemment. Les résultats sont exposés dans le tableau 3 suivant.

**TABLEAU 3**

| Cycles | Concentrations % massiques (% molaire) | | | | Rendement % |
|---|---|---|---|---|---|
| | Glycérol | Carbonate d'éthylène | Carbonate de glycérol | Éthylène glycol | |
| 1 | 8,3 (9,9) | 6,0 (7,5) | 83,3 (78,3) | 2,3 (4,3) | 73,9 |
| 2 | 8,2 (9,9) | 6,5 (8,2) | 82,7 (77,3) | 2,6 (4,7) | 88,6 |
| 3 | 7,8 (9,2) | 6,5 (7,5) | 83,2 (79,6) | 2,5 (3,7) | 90,6 |
| 4 | 7,6 (9,2) | 6,6 (7,8) | 83,5 (78,7) | 2,4 (4,4) | 90,2 |
| 5 | 7,4 (9,0) | 6,6 (7,9) | 83,6 (78,8) | 2,4 (4,3) | 90,3 |
| 6 | 7,5 (8,9) | 6,7 (8,5) | 83,3 (78,2) | 2,4 (4,3) | 89,9 |

## Revendications

1. Procédé de fabrication de carbonate de glycérol à partir de glycérol et d'un carbonate organique cyclique, caractérisé en ce que le glycérol et le carbonate organique cyclique sont amenés à réagir en les mettant en contact dans un milieu solvant essentiellement constitué par un carbonate organique ou un mélange de carbonates organiques, en présence d'un catalyseur solide comprenant une résine macroporeuse anionique bicarbonatée ou hydroxylée, ou une zéolite de type X ou Y tridimensionnelle comportant des sites basiques, le diol formé étant soutiré du milieu au cours de la réaction.

2. Procédé selon la revendication 1, dans lequel on utilise comme solvant un carbonate organique de même nature que le carbonate réactif de départ ou que le carbonate formé, ou bien un mélange de ces deux carbonates.

3. Procédé selon la revendication 2, dans lequel, au début de la réaction, on dispose le glycérol dans un excès de carbonate organique cyclique de façon que ce composé réactif joue initialement le rôle de solvant, le carbonate de glycérol formé faisant ensuite fonction de co-solvant et solvant.

4. Procédé selon l'une des revendications 1 à 3, dans lequel on porte le milieu réactif à une température comprise entre 50° et 110° C.

5. Procédé selon l'une des revendications 1 à 4, mis en oeuvre en continu dans un réacteur ouvert ou une colonne contenant la résine macroporeuse anionique bicarbonatée ou hydroxylée, ou une zéolite de type X ou Y tridimensionnelle comportant des sites basiques, caractérisé en ce que :
- pendant une phase initiale, on délivre dans le réacteur ou la colonne un mélange réactif comportant du carbonate organique cyclique en excès en proportion comprise entre 1,5 et 3 fois la proportion stoechiométrique par rapport au glycérol,
- en sortie de réacteur ou de colonne, on sépare le diol et le carbonate de glycérol formés,
- on réinjecte au moins partiellement le carbonate de glycérol formé dans le réacteur ou la colonne,
- pendant une phase de fonctionnement permanent, on délivre dans le réacteur ou la colonne un mélange réactif comportant le carbonate organique cyclique et le glycérol en proportions sensiblement stoechiométriques, le carbonate de glycérol formé étant toujours au moins partiellement réinjecté dans le réacteur ou la colonne.

6. Procédé selon l'une des revendications 1 à 4, dans lequel les réactifs de départ et le catalyseur solide sont disposés dans un réacteur semi-fermé, avec un excès de carbonate organique cyclique par rapport au glycérol, le diol formé étant soutiré du réacteur au moins pendant la phase d'équilibre de réaction, et du glycérol étant ajouté au cours de la phase d'équilibre de façon à obtenir un épuisement sensiblement concomitant du carbonate cyclique initial et du glycérol.

7. Procédé selon l'une des revendications 1 à 6, dans lequel la fabrication est réalisée à partir de glycérol et de carbonate d'éthylène ou carbonate de propylène.
